# EUROPÄISCHE PATENTANMELDUNG

(11) **EP 1 802 170 A2**
(43) Veröffentlichungstag der Anmeldung: **27.06.2007**
(21) Anmeldenummer: 06123667.5
(22) Anmeldetag: 08.11.2006
(51) Int. Cl.: H04R 25/00

(54) **Verfahren zum Konstruieren einer Otoplastik und zum Einstellen eines Hörgeräts**

(30) Priorität: 22.12.2005 DE 102005061569
(71) Anmelder: Siemens Audiologische Technik GmbH, 91058 Erlangen (DE)
(72) Erfinder: Kasanmascheff, Robert, 91315, Höchstadt (DE)
(74) Vertreter: Berg, Peter

(57) **Zusammenfassung**

Die Anpassung eines Hörgeräts und damit verbunden auch die Formung der Otoplastik oder Hörgeräteschale sollen optimiert werden. Hierfür ist vorgesehen, zunächst einen Teil des Gehörgangs geometrisch zu vermessen (S2, S3), den restlichen Teil des Gehörgangs zu extrapolieren (S5) und daraus ein geometrisches Modell des Gehörgangs zu bilden. Anhand des geometrischen Modells lässt sich sodann ein akustisches Modell des Gehörgangs ermitteln, wobei die Form der Otoplastik oder der Hörgeräteschale in dem akustischen Modell berücksichtigt wird. Die Otoplastik oder Hörgeräteschale lässt sich schließlich mit Hilfe des geometrischen und des akustischen Modells gestalten (S9). Ebenso kann das Hörgerät mit Hilfe des akustischen Modells, das die optimierte Otoplastik/Hörgeräteschale enthält, eingestellt werden.

## Beschreibung

Die vorliegende Erfindung betrifft ein Verfahren zum Konstruieren einer Otoplastik oder einer Hörgeräteschale, wobei ein Teil des Gehörgangs geometrisch vermessen wird. Darüber hinaus betrifft die vorliegende Erfindung ein Verfahren zum Einstellen eines Hörgeräts.

Jeder Gehörgang hat eine bestimmte, charakteristische Akustik. Wird in den Gehörgang beispielsweise eine Otoplastik oder ein In-dem-Ohr-Hörgerät eingesetzt, so verändert sich dessen Akustik.

Hörgeräte wiederum sind an den individuellen Träger anzupassen. Speziell sind sie in Abhängigkeit von dem individuell vorliegenden Gehörgang einzustellen. Hierzu kann der Akustiker beispielsweise Kurven, wie die RECD (real ear to coupler difference), messen, welche dann zu einer verbesserten Anpassung herangezogen werden können. Stehen diesbezüglich Messverfahren nicht zur Verfügung, so werden die Hörgeräte ohne genauere Kenntnisse der individuellen, akustischen Gegebenheiten angepasst.

Da der Gehörgang und die eingesetzte Otoplastik bzw. die Hörgeräteschale in unmittelbarer akustischer Wechselwirkung stehen, sollten die individuellen akustischen Gegebenheiten auch für das Design der Hörgeräteschale bzw. Otoplastik herangezogen werden. Tatsächlich werden die jeweils konkret vorliegenden akustischen Verhältnisse gemessen und in ein Simulationsmodell eingespeist. Das Design der Otoplastik bzw. Hörgeräteschale wird nur aufgrund von Erfahrungswerten beeinflusst. Eine Optimierung des Designs, das ja unmittelbaren Einfluss auf die Akustik hat, ist somit nicht möglich. Aufgrund der Rekursivität dieser Problematik ist eine Einstellung des Hörgeräts so nur bis zu einem gewissen Grad optimierbar.

Aus der Druckschrift EP 0396 831 B1 ist ein Verfahren und ein Gerät zur Bestimmung der akustischen Parameter eines Hörgeräts mit Hilfe eines Softwaremodells bekannt. Dabei werden die Zielhörempfindlichkeit des Benutzers und die Übertragungsfunktion des Hörgeräts bestimmt. Das Softwaremodell der Übertragungsfunktion oder der Übertragungsfunktion eines beispielhaften Modells des Hörgeräts werden gespeichert.

Darüber hinaus zeigt die Druckschrift EP 1 251 716 B1 das Modellieren von Wandlern in einem digitalen Hörgerät. Dabei wird ein elektroakustisches Modell eines digitalen Hörgeräts unter Verwendung eines aufgezeichneten Energiepegels entwickelt.

Ferner ist in der Druckschrift EP 1 207 718 A2 ein Verfahren zur Anpassung eines Hörgeräts beschrieben. Dabei wird ein Modell für die Wahrnehmung einer psycho-akustischen Größe, insbesondere der Lautheit, für eine Norm-Personengruppe parametrisiert. Aufgrund von Modellunterschieden, insbesondere bezüglich ihrer Parametrisierung, werden Stellangaben ermittelt, womit die Signalübertragung an einem Hörgerät ex situ konzipiert oder eingestellt wird beziehungsweise in situ geführt wird.

Die Aufgabe der vorliegenden Erfindung besteht darin, den Konstruktionsvorgang einer Otoplastik oder einer Hörgeräteschale zu verbessern und damit zusammenhängend auch ein optimiertes Einstellungsverfahren für Hörgeräte vorzuschlagen.

Erfindungsgemäß wird diese Aufgabe wird gelöst durch ein Verfahren zum Konstruieren einer Otoplastik oder Hörgeräteschale durch geometrisches Vermessen eines Teils des Gehörgangs, Extrapolieren der Geometrie des restlichen Teils des Gehörgangs, Bilden eines geometrischen Modells aus der gemessenen und der extrapolierten Geometrie des Gehörgangs, Erstellen eines akustischen Modells des Gehörgangs anhand eines geometrischen Modells und Gestalten der Otoplastik oder der Hörgeräteschale mit Hilfe des geometrischen und des akustischen Modells, wobei die Form der Otoplastik oder der Hörgeräteschale in dem akustischen Model berücksichtigt wird.

Weiterhin wird erfindungsgemäß bereitgestellt ein Verfahren zum Einstellen eines Hörgeräts durch geometrisches Vermessen eines Teils des Gehörgangs, Extrapolieren der Geometrie des restlichen Teils des Gehörgangs, Bilden eines geometrischen Modells aus der gemessenen und der extrapolierten Geometrie des Gehörgangs, Erstellen eines akustischen Modells des Gehörgangs anhand des geometrischen Modells, Parametrisieren des akustischen Modells unter Berücksichtigung einer in den Gehörgang eingesetzten Otoplastik oder Hörgeräteschale und Einstellen des Hörgeräts anhand des parametrisierten akustischen Modells.

In vorteilhafter Weise wird erfindungsgemäß beim Konstruieren einer Otoplastik oder Hörgeräteschale die Akustik des Gehörgangs berücksichtigt und darüber hinaus auch beim Einstellen eines Hörgeräts auf die akustischen Gegebenheiten des Gehörgangs Rücksicht genommen.

Vorzugsweise werden die oben genannten Verfahren zur Einstellung eines Hörgeräts und zum Konstruieren einer Otoplastik oder Hörgeräteschale miteinander kombiniert. Dabei wird die Otoplastik oder die Hörgeräteschale mit Hilfe des geometrischen und des akustischen Modells gestaltet und die Form der Otoplastik oder der Hörgeräteschale in dem akustischen Modell berücksichtigt. Dadurch kann sowohl eine optimale Konstruktion der Otoplastik oder Hörgeräteschale als auch eine optimale Einstellung des Hörgeräts erreicht werden.

Beim Vermessen der Geometrie des Teils des Gehörgangs kann ein aus der algorithmischen Geometrie bekanntes Skelett, eine Mittenlinie, ein Oberflächenverlauf und/oder ein Volumenverlauf des Gehörgangs ermittelt werden und diese Daten können anschließend für die Extrapolation verwendet werden. Für das Vermessen des Gehörgangs können somit vorteilhaft der Methoden der algorithmischen Geometrie eingesetzt werden.

In dem vorliegenden Dokument wird unter Mittenlinie ein Linienzug oder ein Baum von Linien verstanden, welcher Mitteneigenschaften bezüglich des Skeletts besitzt. Die Berechnung von Mittenlinien erfordert nicht notwendigerweise die Berechnung eines Skeletts. Eine Mittenlinie ist eine Submenge der Punkte einer Mittenachse. Die Mittenachse eines Körpers (z. B. des Gehörgangs wird durch die Menge der Mittelpunkte aller Kugeln beschrieben, welche die Oberfläche des Körpers an mindestens zwei Punkten berühren, die Oberfläche aber nicht schneiden.

Bei einer speziellen Ausgestaltung der erfindungsgemäßen Verfahren kann das Vermessen des Gehörgangs durch direktes scannen erfolgen. Dies ist zwar aparativ sehr aufwändig, liefert jedoch im Idealfall die besten Ergebnisse. Außerdem sind keine zeitintensiven Zwischenschritte erforderlich.

Alternativ kann zum Vermessen des Gehörgangs auch ein Ohrabdruck genommen und dieser gescannt werden. Ein derartiges Messverfahren ist verhältnismäßig einfach realisierbar.

Bei einer weiteren vorteilhaften Ausgestaltung des erfindungsgemäßen Verfahrens wird beim Extrapolieren das Skelett oder die Mittenlinie des restlichen Teils des Gehörgangs extrapoliert. Aus diesen Daten kann dann der Oberflächenverlauf des Gehörgangs einfacher extrapoliert werden.

Das Parametrisieren des akustischen Modells einerseits und das Gestalten der Otoplastik oder Hörgeräteschale bzw. das Einstellen des Hörgeräts andererseits kann zur Optimierung mehrfach abwechselnd wiederholt werden. Auf diese Weise kann man sehr rasch zu einer optimalen Otoplastik und einer optimalen Einstellung des Hörgeräts gelangen.

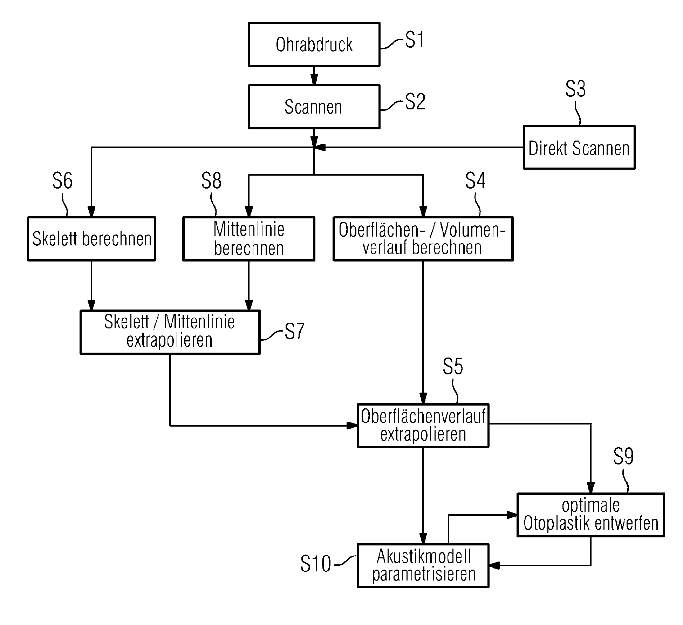

Die vorliegende Erfindung wird nun anhand der beigefügten Zeichnung näher erläutert, die ein Ablaufschema für mehrere erfindungsgemäße Verfahrensvarianten zeigt.

Die nachfolgend näher geschilderten Ausführungsbeispiele stellen bevorzugte Ausführungsformen der vorliegenden Erfindungen da.

Zur optimierten Gestaltung einer Otoplastik und zum optimalen Einstellen des entsprechenden Hörgeräts wird gemäß der Figur zunächst in einem Schritt S1 ein Ohrabdruck eines Teils des Gehörgangs genommen. Dieser Ohrabdruck wird in Schritt S2 gescannt, um seine Oberflächendaten zu erhalten. Diese Oberflächendaten können entsprechend Schritt S3 auch direkt gewonnen werden, indem das Ohr bzw. der Gehörgang direkt abgescannt wird. In jedem Fall beziehen sich die gescannten Daten nur auf einen Teil des Gehörgangs. Sinn ist es nun, aus diesem nun bekannten Teil des Gehörgangs Daten für die Geometrie des gesamten Gehörgangs zu gewinnen. Dazu kann gemäß Schritt S4 aus den Scanndaten direkt der Oberflächen- oder Volumenverlauf des bekannten Teils des Gehörgangs berechnet und daraus entsprechend Schritt S5 der Oberflächenverlauf des gesamten Gehörgangs extrapoliert werden. Die Extrapolation des gesamten Oberflächenverlaufs (Schritt S5) aus den berechneten Oberflächendaten des bekannten Teils des Gehörgangs ist jedoch sehr rechenaufwändig.

Alternativ wird aus den gescannten Daten des bekannten Teils des Gehörgangs aus Schritt S2 ein sogenanntes Skelett berechnet (Schritt S6). Unter dem Skelett wird eine geometrische Struktur verstanden, wie dies in der algorithmischen Geometrie geläufig ist. Skelette können für dreidimensionale Gebilde aber auch für Flächen ermittelt werden. Für die vorliegende Anwendung ist es jedoch nicht zwingend erforderlich, das Skelett exakt zu berechnen, es kann auch eine ähnliche geometrische Struktur ermittelt werden.

Aus den Skelettdaten des bekannten Teils des Gehörgangs lässt sich ohne großen Aufwand das Skelett des restlichen Teils des Gehörgangs extrapolieren (Schritt S7). Aus dem Skelett des gesamten Gehörgangs lässt sich dann ohne weiteres der Oberflächenverlauf des Gehörgangs ermitteln (Schritt S5).

Anstelle des Skeletts kann auch eine Mittenlinie aus den Scanndaten des bekannten Teils des Gehörgangs gemäß Schritt S8 berechnet werden. Diese Mittenlinie des bekannten Teils des Gehörgangs lässt sich wie das Skelett in einfacher Weise entsprechend Schritt S7 extrapolieren. Wiederum wird aus der extrapolierten Mittenlinie in Schritt S5 der Oberflächenverlauf des unbekannten Teils des Gehörgangs extrapoliert. Wahlweise ist also eine Extrapolation mit wenig Rechenaufwand über das Skelett oder die Mittenlinie möglich. Etwas mehr Rechenaufwand erfordert, wie bereits erwähnt, die Extrapolation direkt über die Oberflächendaten.

Aus den Oberflächendaten kann einerseits gemäß Schritt S9 eine Otoplastik entworfen werden und andererseits ein Akustikmodell entsprechend Schritt S10 parametrisiert werden. In dem akustischen Modell kann die eingesetzte Otoplastik mit berücksichtigt werden, was in der FIG der Pfeil vom Schritt S9 zu Schritt S10 symbolisiert. Umgekehrt lässt mit dem akustischen Modell und dem geometrischen Modell, d. h. den Oberflächendaten, die Otoplastik beziehungsweise Hörgerätschale optimieren. Dies ist in der FIG durch den Pfeil von Schritt S10 zu Schritt S9 angedeutet. Zwischen den Schritten S9 und S10 ergibt sich somit eine Schleife, die zur Optimierung des Akustikmodells bzw. der Otoplastik mehrfach durchlaufen werden kann. Letztlich ergibt sich eine akustische optimale Otoplastik oder Hörgeräteschale und ein individuell optimiertes Akustikmodell, das als Basis für die Einstellung des Hörgeräts dienen kann.

Aufgrund der erfindungsgemäßen Extrapolation und der darauf basierenden Parametrisierung des Akustikmodells beziehungsweise Optimierung der Otoplastik muss der Akustiker keine aufwändigen Messungen mehr durchführen, um eine relativ genaue Anpassung durchführen zu können.

Ferner kann die Geometrie der Hörgeräteschale oder Otoplastik akustisch optimal erstellt werden. Schließlich kann man Fehlern in der Anpassstrategie leichter auf den Grund gehen, da die simulierten Kurven besser die Realität wiedergeben.

## Patentansprüche

1. Verfahren zum Konstruieren einer Otoplastik oder Hörgeräteschale durch
- geometrisches Vermessen (S2, S3) eines Teils eines Gehörgangs, Extrapolieren der Geometrie des restlichen Teils des Gehörgangs,
- Bilden (S4, S6, S7, S8) eines geometrischen Modells aus der gemessenen und der extrapolierten Geometrie des Gehörgangs,
- Erstellen eines akustischen Modells des Gehörgangs anhand eines geometrischen Modells und
- Gestalten (S9) der Otoplastik oder der Hörgeräteschale mit Hilfe des geometrischen und des akustischen Modells, wobei die Form der Otoplastik oder der Hörgeräteschale in dem akustischen Modell berücksichtigt wird.

2. Verfahren zum Einstellen eines Hörgeräts durch
- geometrisches Vermessen (S2, S3) eines Teils des Gehörgangs,
- Extrapolieren der Geometrie des restlichen Teils des Gehörgangs,
- Bilden (S4, S6, S7, S8) eines geometrischen Modells aus der gemessenen und der extrapolierten Geometrie des Gehörgangs,
- Erstellen eines akustischen Modells des Gehörgangs anhand eines geometrischen Modells,
- Parametrisieren (S 10)des akustischen Modells unter Berücksichtigung einer in den Gehörgang eingesetzten Otoplastik oder Hörgeräteschale und
- Einstellen des Hörgeräts anhand des parametrisierten akustischen Modells.

3. Verfahren nach Anspruch 2, wobei die Otoplastik oder die Hörgeräteschale mit Hilfe des geometrischen und des akustischen Modells gestaltet wird (S9) und dabei die Form der Otoplastik oder der Hörgeräteschale in dem akustischen Modell berücksichtigt wird.

4. Verfahren nach einem der vorhergehenden Ansprüche, wobei beim Vermessen der Geometrie des Teils des Gehörgangs eine Skelett (S6), eine Mittenlinie (S8), ein Oberflächenverlauf und/oder ein Volumenverlauf (S4) des Gehörgangs ermittelt wird und diese Daten für die Extrapolation verwendet werden.

5. Verfahren nach einem der vorhergehenden Ansprüche, wobei beim Vermessen der Gehörgang direkt gescannt wird (S3).

6. Verfahren nach einem der Ansprüche 1 bis 4, wobei zum Vermessen des Gehörgangs ein Ohrabdruck (S1) genommen und dieser gescannt (S2) wird.

7. Verfahren nach einem der Ansprüche 4 bis 6, wobei beim Extrapolieren das Skelett oder die Mittenlinie des restlichen Teils des Gehörgangs extrapoliert (S7) wird.

8. Verfahren nach einem der vorhergehenden Ansprüche, wobei das Parametrisieren des akustischen Modells (S10) einerseits und das Gestalten der Otoplastik oder Hörgeräteschale (S9) bzw. das Einstellen des Hörgeräts andererseits zur Optimierung mehrfach abwechselnd wiederholt wird.
